# EUROPEAN PATENT APPLICATION

(11) **EP 1 872 733 A1**
(43) Date of publication of application: **02.01.2008**
(21) Application number: 07252572.8
(22) Date of filing: 25.06.2007
(51) Int. Cl.: A61B 17/88, A61B 17/00, A61B 17/74

(54) **Bone plate clamp**

(30) Priority: 27.06.2006 US 426606
(71) Applicant: DePuy Products, Inc., Warsaw, IN 46581 (US)
(72) Inventor: Orbay, Jorge L., Coral Gables, FL 33156 (US); Sixto, Robert Jr., Miami, FL 33156 (US); Cavallazzi, Cesare, Miramar, FL 33027 (US); Francese, Jose Luis, Miami Springs, FL 33166 (US)
(74) Representative: Belcher, Simon James

(57) **Abstract**

A bone plate clamp includes a C-shaped frame with opposing arms spaced apart about a bridge portion. One arm includes a threaded bore into which an adjustable clamping screw having a clamping end is inserted. The clamping screw defines a longitudinal drill guide. The opposite arm has a V-shaped surface oriented to support the bone at two points on the opposite side of the bone from the clamping end of the screw such that the bone clamp securely holds the bone and the bone plate together at three points. The bridge portion is structured to flex when the clamp applies a compressive force between the first and second arms. Flexing of the bridge applies a preload to the clamp which allows the clamp to function as a spring and maintain compression over a range of bone-plate dimensions. The bone plate clamp also includes a removable handle.

## Description

This invention relates broadly to surgical instruments. In particular this invention relates to clamps used in surgical orthopaedic procedures, and more particularly to clamps used to hold bone plates against bones during surgical procedures.

During surgical orthopaedic procedures in which a plate is attached to a bone, it is necessary to fix the plate to the bone after fracture reduction so that holes may be drilled for the plate fasteners; e.g., bone screws. The surgeon may insert a bone screw to hold the plate at the anticipated implant location. However, if there is a need for adjustment, the screw may need to be removed, the screw hole re-drilled, and the screw inserted. The process is repeated until plate location is satisfactory. If the plate has to be relocated, which can involve forming a new screw hole, the underlying bone can be compromised.

Clamping is commonly used to provide temporary fixation without compromising the bone. The clamps are generally either forceps-style clamps or C-clamps.

US-5797919 discloses a forceps-style clamp which has two clamping surfaces mounted on arms coupled relative to a pivot point. One significant disadvantage of such clamps is that the arms and handle of the instrument approach the surgical wound transverse the direction of the clamping force. Thus, positioning the clamping surfaces around the bone is difficult and the surgical wound may need to be opened up more than necessary for clamp access. In addition, given the transverse extension of the handle, there is significant opportunity for the handle to be bumped by the surgeon during the procedure or even for the tissue surrounding the surgical wound to apply sufficient force to the handle to cause inadvertent movement of the clamp and move the plate relative to the bone.

US-4187840 discloses a C-clamp which has clamping surfaces that longitudinally translate relative to each other, rather than pivot relative to each other. The handle extends up and out of the wound rather than transverse to it. However, the upwardly extending handle remains in the way of the surgeon, obstructing the portion of the plate held by the arms and limiting access for drilling K-wires and holes in the plate. The clamp has two clamping surfaces that longitudinally translate relative to each other. It is a stiff design adapted to apply an axial clamping force via rotation of a leadscrew. Once the leadscrew is set, the clamp has a very small dimensional tolerance over which it will maintain compression. However, it is appreciated by the inventors that long bones are not perfect cylinders and that such bones change in diameter along their length. Thus, when this type of clamp is holding a plate to a long bone and the two clamping arms are set a distance apart sufficient to apply a clamping force, even a small inadvertent movement of the clamp and plate along the long bone may result in an undesirable change in bone diameter which can provides additional space between the clamping arms for the bone and plate. Such results in a loss of compression and complete loss of retention of the plate to the bone.

The present invention provides a bone plate clamp for temporary fixation of a bone plate on a bone is provided. The clamp includes a generally C-shaped frame having first and second opposing arms spaced apart about a bridge portion of the frame. The arms are configured to surround a portion of bone when the arms are positioned transversely with respect to the longitudinal axis of the bone.

Preferably, the first arm of the frame includes a threaded bore into which an adjustable clamping screw having a clamping end is inserted. The clamping screw has a longitudinal axial bore sized for guiding a drill bit and which functions as drill guide. The head of the clamping screw includes recesses to maximize clearance for drilling K-wires about the clamping screw into a bone plate held by the clamp.

Preferably, the second arm has a V-shaped surface oriented to support the bone at two points on the opposite side of the bone from the clamping end of the screw such that the bone clamp securely holds the bone and the bone plate together at three points. The second arm preferably has an opening positioned at the apex of the V-shaped surface. The opening is sized and aligned for passage of a drill bit which may extend through the bore in the clamping screw. As described in more detail below, the opening is preferably oblong to accommodate passage of the drill bit even when the bridge portion flexes and deforms in shape when the frame of the clamp is subject to a pre-load.

Preferably, the bridge portion is structured to flex when the clamp applies a predetermined compressive force between the first and second arms. Flexing of the bridge applies a preload to the frame which allows the frame to function as a spring and the clamp to maintain compression over a range of bone-plate dimensions. That is, if the clamp is inadvertently contacted such that the clamp and plate are moved along the bone a small distance but a distance which corresponds to a change in diameter of the bone, the clamp will maintain compression of the plate to the bone at the new location.

The bone clamp may also include a handle removably coupled to the frame. The handle can be quickly disassembled during surgery to provide increased visual and physical access to the surgical site. In addition, such allows manipulation of the arm under fluoroscopy with minimal inconvenience. Furthermore, the handle can also be quickly assembled to the clamp during surgery in a manner that provides strong and rigid mechanical coupling with the clamp, e.g., to facilitate movement of the clamp (and plate) along the bone.

Preferably, the threaded bore and clamping screw coupling can be replaced with mechanisms providing quick compression of the plate to the bone.

The bone plate clamp of the invention can provide compression of a bone plate along a bone which may change shape and dimension along its length.

The bone plate clamp of the invention can be conveniently applied to the bone and plate.

The bone plate clamp of the invention can be manoeuvred conveniently along the bone.

The bone plate clamp of the invention can have a low profile at the surgical wound during use.

The bone plate clamp of the invention can have additional functionality beyond clamping.

The bone plate clamp of the invention can be convenient to manufacture.

The device of the invention can be used in a method of implanting a plate on a bone fracture, comprising:
(a) positioning a plate over a reduced bone fracture;
(b) clamping the plate to the bone with a bone plate clamp, the plate having a screw hole and the clamp having a drill guide positioned at the screw hole;
(c) drilling a hole through the drill guide and the screw hole and into the bone;
(d) releasing the clamp; and
(e) inserting a screw into the screw hole.

Embodiments of the invention will now be described by way of example with reference to the accompanying drawings, in which:
Fig. 1 is a side elevation of a bone plate clamp according to the invention, shown with a long bone and plate in section view;
Fig. 2 is an exploded perspective view of the bone plate clamp of Fig. 1;
Fig. 3 a perspective view of the bone plate clamp, with the handle shown in a broken view, shown relative to a bone plate and K-wire;
Fig. 4 is a perspective view of a first alternative embodiment of a frame element of a bone plate clamp according to the invention;
Fig. 5 is an enlarged partial section view of a portion of an upper arm of the frame element of Fig. 4; and
Fig. 6 is a perspective view of a second alternative embodiment of a frame element of a bone plate clamp according to the invention.

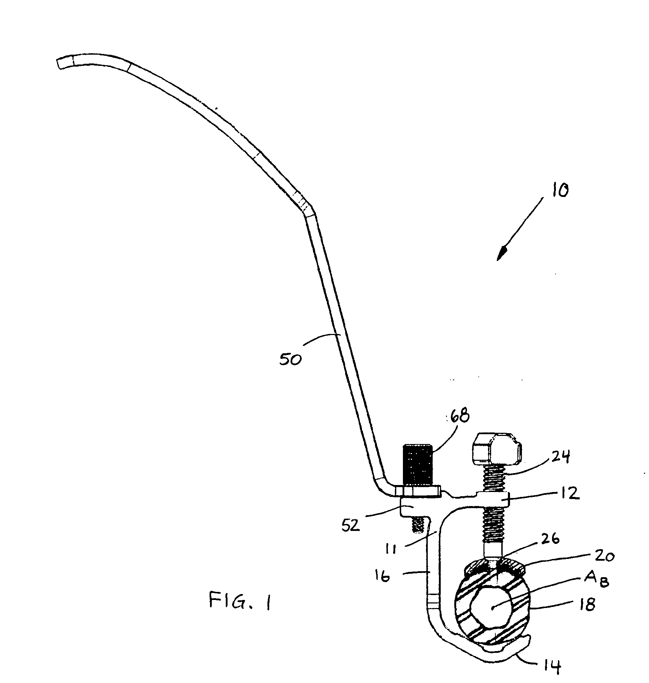

Referring to the drawings, Fig. 1 shows a bone plate clamp 10 for temporary fixation of a bone plate on a fractured bone is provided. The clamp 10 includes a C-shaped frame 11 having first and second opposed, preferably stationary arms 12, 14 spaced apart about a bridge portion 16 of the frame. The arms 12, 14 are configured to surround a portion of bone 18 and a plate 20 when the arms are positioned transversely with respect to the longitudinal axis AB of the bone.

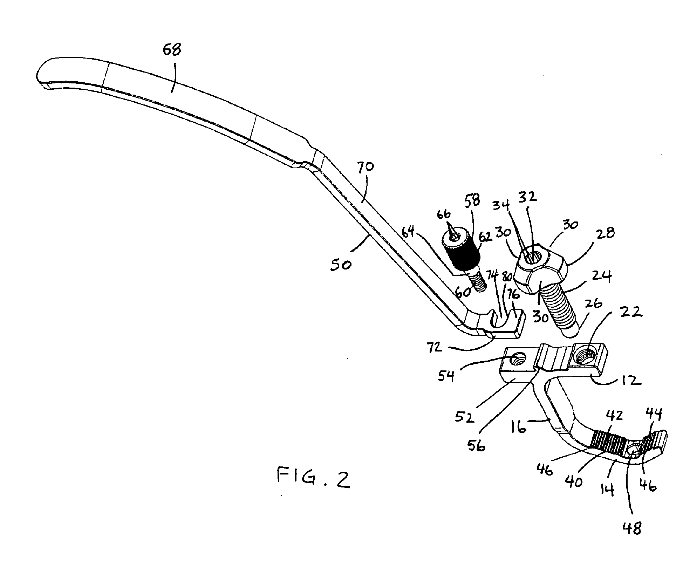

Referring to Figs. 1 and 2, the first arm 12 of the clamp include a threaded bore 22 into which an adjustable clamping screw 24 is inserted. The clamping screw has a clamping end 26 and a head 28. The clamping end is sized to seat partially within a screw hole on the bone plate 20. The head 28 is provided with circumferentially spaced apart recesses or scallops 30 to maximize clearance for drilling K-wires about the clamping screw into a bone plate held by the clamp, as described in more detail below. The clamping screw also has a longitudinal axial bore 32 sized for guiding a drill bit. The head end of the bore 32 is provided with driver engagement means, such as corners 34 for engaging a hex driver.

The second arm 14 has a V-shaped clamping surface 40 oriented to support the bone at two points 42, 44 on the opposite side of the bone from the clamping end 26 of the clamping screw 24 such that the bone clamp securely holds the bone and the bone plate together at three points. The surface 40, preferably at least at points 42, 44 is provided with a bone engaging structure such as teeth or ridges 46. An opening 48 is positioned at the apex of the V-shaped clamping surface 40. The opening 48 is preferably oblong (longer in the direction in which the second arm 14 extends) and sized for passage of a drill bit that may be extended through the axial bore 32 in the clamping screw 24. As described in more detail below, the opening 48 is preferably oblong to accommodate passage of the drill bit even when the budge portion 16 flexes when the frame 11 is subject to a pre-load, as now described.

The bridge portion 16 is structured by material and dimension to flex when the clamp 10 applies a compressive force between the first and second arms 12, 14; i.e., by axial movement of the clamping end 26 of the clamping screw 24 toward the second arm 14 to apply a force against the bone 18 and the plate 20 sufficient to deform the bridge portion 16. Such flexing of the bridge portion 16 applies a preload that allows the frame 11 to function as a spring and the clamp 10 to maintain compression over a range of bone-plate dimensions. By way of example only, the bridge portion 16 may flex approximately 2° to 3°, and that may correspond to an approximately 2 mm change in the distance between the first and second arms 12, 14. Accordingly, if the clamp 10 is inadvertently moved such that the clamp 10 and plate 20 are turn moved along the bone a small distance in which the bone decreases in diameter up to 2 mm, the clamp will maintain compression on the bone and the plate at the new location. It is appreciated that adjustment of the material and/or dimension of the bridge portion 16 operates to adjust the spring rate of the clamp. The geometrical cross-sectional of the bridge portion 16 is such that the force applied by the clamping screw to induce the stress sufficient for the above described flexing is less than the yield strength of the material prior to the clamping screw bottoming out. In view of the above, the clamp 10 is preferably adapted to apply a force of from about 133 to about 444 N (30 to 100 lb.f).

As indicated above, the opening 48 is oblong. The oblong configuration of opening 48 allows at least a portion of the opening to remain in alignment with the longitudinal bore 32 of the clamping screw 24 for passage of a drill even after the bridge portion 16 flexes by up to several degrees.

The bone clamp 10 may also include a handle 50 removably coupled to the frame 11. The frame 11 preferably includes handle mount 52 at the upper end of the bridge portion 16 extending opposite the first arm 12. The mount 52 includes a threaded bore 54 and a flat stop 56. A set screw 58 is provided in the threaded bore 54 for coupling the handle 50 to the mount 52 and preferably remains coupled to mount 54 at the bore 54 even when the handle 50 is decoupled. The set screw 58 includes a lower threaded shank 60, an upper knob 62 and an shoulder 64 between them. The knob 62 is preferably provided with driver engagement means, such as corners 66 in a hex arrangement. The handle 50 includes a gripping end 68, a shaft portion 70 and a coupling end 72. Coupling end 72 includes a lateral slot 74 having an angled side wall 80, and a rear edge 76 spaced a fixed distance from the slot 74. In assembly, the set screw is provided in the threaded bore with the bottom of the shoulder 64 substantially level with the top surface of the mount 52. The slot 74 of the coupling end 72 of the handle 50 is positioned about the shoulder 64, with the edge 76 located between the shoulder 64 and the stop 56 and the shoulder 64 contacting the angled wall 80 of the slot 74. This configuration accommodates a range of part tolerances and assures a tight fit. When the knob 62 is rotated and tightened down on the coupling end 72, the handle 50 is rigidly held relative to the frame 11. A driver may be coupled at driver engagement means 66 for final tightening. The handle 50 can be used to facilitate placement of the clamp, movement of the clamp and plate along the bone after the clamping screw is partially located within a screw hole but before a clamping force is applied, and removal of the clamp from the surgical site. However, during the surgical procedure the handle can be quickly disassembled to provide increased visual and physical access to the surgical site. In addition, removal of the handle such allows manipulation of the arm under fluoroscopy with minimal inconvenience.

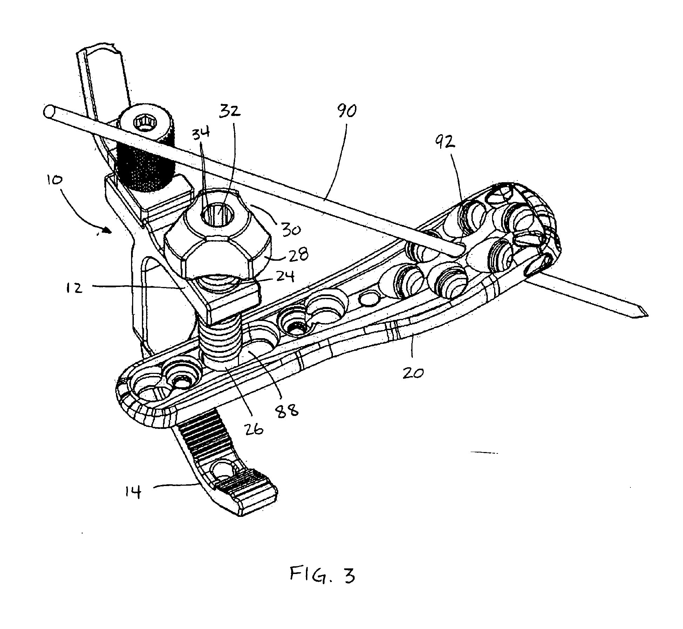

One method of using the clamp is now described with reference to Fig. 3. The fracture site is accessed and debrided, and the fracture is reduced. A bone plate 20 is then located at the fracture site. The first and second arms 12, 14 of the clamp 10 are located about the fracture of the bone, with the first arm 12 positioned over the plate 20. The clamping end 26 of the clamping screw 24 is located partially in one of the screw holes 88 of the plate 20, but diametrically sized such that it cannot extend all the way through the hole. It is preferred that the screw hole 88 be a non-fixed angle screw hole, as the longitudinal bore 32 of the clamping screw 24 will be used as a drill guide through the screw hole 88, as described hereafter, and perfect axial alignment between the longitudinal bore and the screw hole is difficult to assure given that the bone is not a perfect cylinder. The handle 50 may be used to manoeuvre the plate 20 along the bone. The plate 20 position is verified by the surgeon and the clamp 10 is then tightened down on the plate as described above. If desired, a manual driver can be coupled to the driver engagement means 34 of the clamping screw to facilitate tightening of the clamping screw 24. Once the clamp 10 is tightened, the handle 50 is optionally removed. (It is appreciated that use and/or provision of the handle 50 entirely optional, and also that it may remain coupled to the frame 11 throughout the procedure, both options at the discretion of the manufacturer and/or surgeon.)

A K-wire 90 may be drilled through a preferably strategically located fixed angle K-wire hole 92 in the plate 20 and into the underlying bone (not shown) to stabilize the fracture reduction. As indicated above, a recess 30 in the head 28 of the clamping screw 24 provides additional clearance for the K-wire. The K-wire 90 holds the plate 20 over the fracture at the location where the surgeon has anticipated it should be implanted. The plate location is then confirmed by viewing the location of the plate and the K-wire relative to the bone under fluoroscopy. If indicated by fluoroscopy, the plate 20 can be repositioned to a new location by removing the K-wire 90, loosening the clamp 10, and the repositioning the plate 20. The K-wire can then be redrilled and the new location again confirmed relative to the anatomy under fluoroscopy. The use of K-wires through strategically located fixed angle holes in a plate is disclosed in US-A-2005/0065524 and US-A-2005/0182406.

Once the location is approved by the surgeon, a hole is drilled through the longitudinal bore 32 of the clamping screw 24 and the aligned bone screw hole 88 in the plate. The clamp 10 is then removed and a screw (not shown) is inserted through screw hole 88 in the plate and into the bone. With the plate fixed relative to the bone, the remaining screws and other fasteners, if any, are then inserted through the plate and into the bone in a conventional manner.

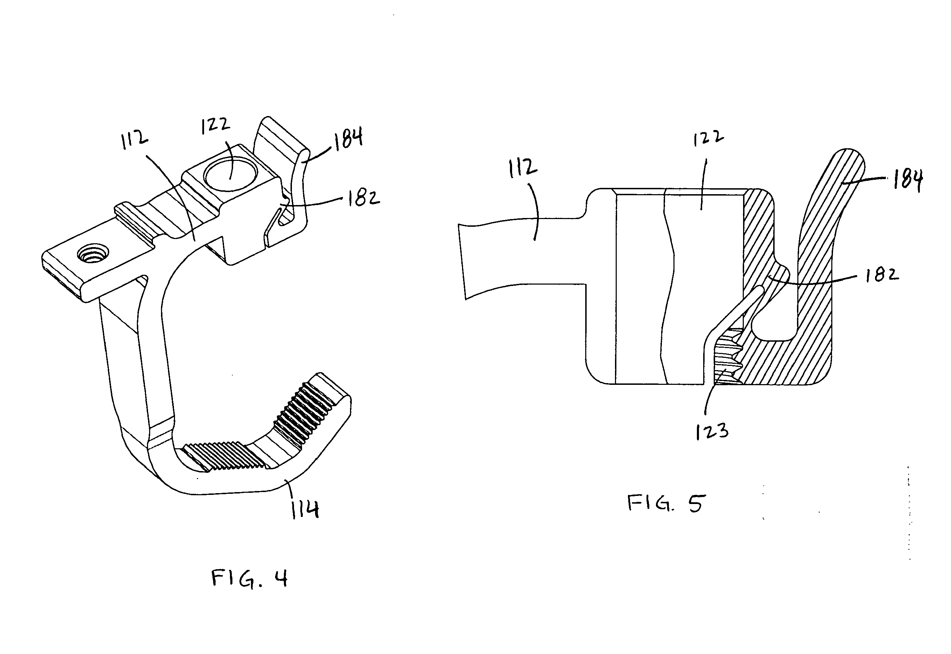

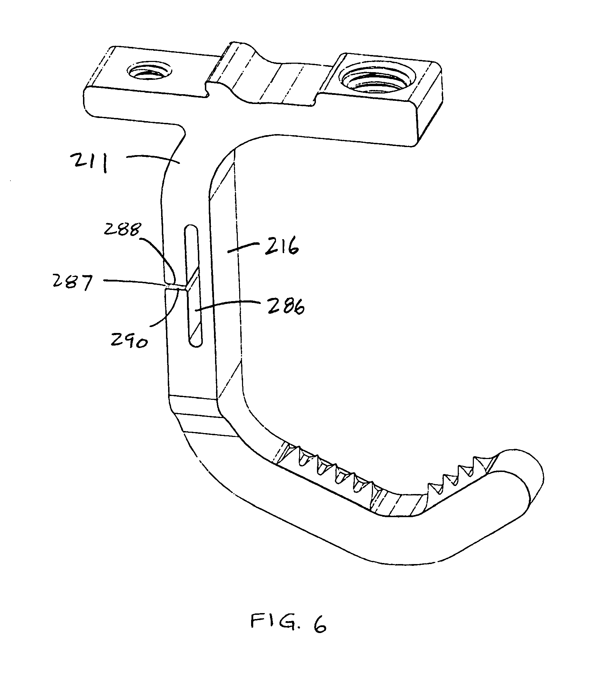

It can be desirable to facilitate quickly dropping the clamping end of the clamping screw on to the plate (as opposed to rotating the clamping screw all the way down) from a clamp-release position into an initial plate contact position prior to applying a clamping force. Turning now to Figs. 4 and 5, one exemplar embodiment for rapidly moving the clamping screw between the clamp-release and initial plate contact positions is shown. The embodiment includes a live hinge 182 at an outer side of the longitudinal bore 122 of the first arm 112. Threads 123 are provided at a lower end of the bore 122 below the hinge 182. A lever 184 is coupled to the hinge 182 and can be actuated to increase the diameter of the bore to allow the clamping screw 24 (Figs. 1 and 2) to fall through the bore 122 until the lever 184 is released. Once released, the clamp with clamping screw 24 are operated as described above. It is also appreciated that even other means to quickly move the clamping screw or another force applying element (for example, a rod or a bolt) from a clamp-release position into a contact position can also be used, such as an over-centre toggle clamp. Fig. 4 also shows that the second arm 114 is not necessarily provided with an opening at the apex of the arm.

In addition, while the bridge portion of the frame of the clamp is adapted to flex for pre-load by controlling its dimensions and materials, Fig. 6, shows another embodiment of a frame 211 for a clamp. Frame 211 has a bridge portion 216 with a longitudinal slot 286 and an entry 287 into the slot having generally opposed surfaces 288, 290 angled relative to each other by approximately 2° to 3°. When a clamp with frame 211 is operated to apply a force, i.e., by axial displacement of the clamping screw (not shown), the bridge 216 will deform to move the opposed surfaces 288, 290 toward and then into contact with each other. Once they are in contact with each other, the surface-to-surface contact functions as a stop, signalling to the surgeon that the maximum recommended pre-load has been applied to the clamp and that the clamp should not be tightened further.

The clamp and handle are preferably constructed of stainless steel, with the clamp made from 17-4 stainless steel for its mechanical properties. The clamp and handle may be constructed by electrical discharge machining (EDM), water jet machining, or laser cutting, among other means. As such, the clamp is relatively easy and inexpensive to manufacture.

While particular dimensional tolerances have been related to the angular displacement of the bridge portion under pre-load, it is recognized that the actual dimensional tolerances provided by a particular angular displacement will be related to the overall size of the frame and that clamps of different sizes may be able to accommodate a different tolerance for dimensional change under pre-load. In addition, the clamp frame and handle may be constructed of materials other than those described.

## Claims

1. A bone plate clamp for use on a bone plate on a bone having a longitudinal axis, the clamp comprising:
(a) a frame configured to at least partially surround a portion of the bone when the frame is positioned transversely with respect to the longitudinal axis of the bone; and
(b) force applying means movable relative to the frame,
in which a deformable portion of the frame deforms when a predetermined load is applied to the plate and the bone such that the clamp applies a pre-load to the plate and bone such that the clamp can maintain compression over a range of bone-plate dimensions.

2. A clamp according to claim 1, in which deformable portion flexes up to approximately 3°.

3. A clamp according to claim 1, in which the frame includes first and second arms spaced apart relative to a bridge portion, and the deformable portion of the frame is the bridge portion.

4. A clamp according to claim 3, in which the bridge portion is made of a material, and the bridge portion has a geometrical cross-sectional such that the force applied by the force applying element to induces a stress sufficient to flex the bridge portion is less than the yield strength of the material.

5. A clamp according to claim 3, in which the bridge portion includes a longitudinal slot and an entry into the slot having generally opposed surfaces, wherein when the force applying element is operated to apply a force to preload the frame, the bridge deforms to move the opposed surfaces toward each other.

6. A clamp according to claim 5, in which the opposed surfaces are angled relative to each other.

7. A clamp according to claim 6, in which the opposed surfaces contact each other to operate as a stop when a predetermined pre-load has been applied to the frame.

8. A clamp according to claim 3, in which the second arm is V-shaped.

9. A clamp according to claim 1, in which the force applying element includes a drill guide.

10. A clamp according to claim 9, in which the frame includes first and second arms spaced apart, the force applying element is coupled to and movable relative to the first arm, and the second arm includes an opening in alignment with the drill guide when the frame is subject to a pre-load.

11. A clamp according to claim 10, in which the opening is oblong.

12. A clamp according to claim 1, in which the force applying element is a screw.

13. A clamp according to claim 12, in which the screw includes a head with circumferentially displaced recesses.

14. A clamp according to claim 1, which includes a handle coupled to the frame.

15. A clamp according to claim 14, in which the handle is removably coupled to the frame.

16. A clamp according to claim 1, in which the frame is generally C-shaped.

17. A clamp according to claim 1, which includes a component for moving a clamping end of the force applying element from a clamp-release position into an initial plate contact position.

18. A bone plate clamp for use on a bone plate on a bone having a longitudinal axis, the clamp comprising:
(a) a frame configured to at least partially surround a portion of the bone when the frame is positioned transversely with respect to the longitudinal axis of the bone; and
(b) a force applying element movable relative to the first arm, the force applying element including a drill guide.

19. A clamp according to claim 18, in which the force applying element is a screw and the drill guide is a longitudinal bore through the screw.

20. A clamp according to claim 19, in which the screw includes a head with circumferentially displaced recesses.

21. A clamp according to claim 18, in which the frame includes first and second arms spaced apart relative to a bridge portion.

22. A clamp according to claim 21, in which the second arm includes an opening in alignment with the drill guide.

23. A clamp according to claim 18, which includes a handle coupled to the frame.

24. A clamp according to claim 18, which includes a component for rapidly moving a clamping end of the force applying element from a clamp-release position into an initial plate contact position.
